# EUROPEAN PATENT SPECIFICATION

(11) **EP 4 234 803 B1**
(45) Date of publication and mention of the grant of the patent: **09.12.2026**
(21) Application number: 23176859.9
(22) Date of filing: 13.02.2018
(51) Int. Cl.: D06M 13/17, D06M 13/203, D06M 15/03

(54) **ANTI-ODOR COMPOSITIONS, STRUCTURES HAVING ANTI-ODOR CHARACTERISTICS, METHODS OF MAKING THE ANTI-ODOR COMPOSITIONS AND THE STRUCTURES**
GERUCHSHEMMENDE ZUSAMMENSETZUNGEN, STRUKTUREN MIT GERUCHSHEMMENDEN EIGENSCHAFTEN, VERFAHREN ZUR HERSTELLUNG DER GERUCHSHEMMENDEN EIGENSCHAFTEN UND STRUKTUREN
COMPOSITIONS ANTI-ODEURS, STRUCTURES AYANT DES CARACTÉRISTIQUES ANTI-ODEURS, PROCÉDÉS DE FABRICATION DES COMPOSITIONS ANTI-ODEURS ET DES STRUCTURES

(30) Priority: 14.02.2017 US 201762458616 P; 14.02.2017 US 201762458632 P
(43) Date of publication of application: 30.08.2023
(62) Document number(s) of the earlier application(s) in accordance with Art. 76 EPC: 20187555.6 / 3 748 067; 18707543.7 / 3 568 518
(73) Proprietor: NIKE Innovate C.V., Beaverton, OR 97005 (US)
(72) Inventor: HU, Cheng, Beaverton, OR 97005 (US)
(74) Representative: Müller-Boré & Partner Patentanwälte PartG mbB

(56) References cited:
- EP-A2- 1 129 693
- JP-A- 2010 132 634
- US-A1- 2007 105 745
- US-B1- 6 689 378

## Description

### CROSS-REFERENCE TO RELATED APPLICATIONS

This application claims the benefit of and priority to U.S. Provisional Application Serial No. 62/458,616, having the title "ANTI-ODOR COMPOSITIONS, STRUCTURES HAVING ANTI-ODOR CHARACTERISTICS, METHODS OF MAKING THE ANTI-ODOR COMPOSITIONS AND THE STRUCTURES", filed on February 14, 2017; and this application also claims the benefit of and priority to U.S. Provisional Application Serial No. 62/458,632, having the title "ANTI-ODOR COMPOSITIONS, STRUCTURES HAVING ANTI-ODOR CHARACTERISTICS, METHODS OF MAKING THE ANTI-ODOR COMPOSITIONS AND THE STRUCTURES", filed on February 14, 2017.

### BACKGROUND

During exercise or strenuous effort, humans perspire and the perspiration is absorbed by the clothing. The components of the perspiration can be converted to malodorous compounds through chemical or biochemical mechanisms that can involve naturally present bacteria on the skin. In addition, compounds on the clothing can react during cleaning and cause odors that are difficult to remove from the clothing. There are many ways to address the odor and some include masking the odor with more pleasant smells, prevent the odor from being formed by wicking the perspiration from the skin, killing the bacteria that can produce the malodorous compounds, and the like. However, these efforts have not addressed all of the problems associated with odors and may cause other problems.

Document JP 2010 132634 A relates to a deodorant that can be used for fiber treatment, that comprises trehalose.

### BRIEF DESCRIPTION OF THE DRAWINGS

Further aspects of the present disclosure will be more readily appreciated upon review of the detailed description of its various embodiments, described below, when taken in conjunction with the accompanying drawings.
Figure 1 is an example of an article of apparel with anti-odor characteristics.
Figures 2A-G illustrates graphs of gas chromatograph/gas chromatograph-time of flight mass spectrometry (GC/GC-TOFMS) profiling of aldehyde compounds on worn fabric samples.

The drawings illustrate only example embodiments and are therefore not to be considered limiting of the scope described herein, as other equally effective embodiments are within the scope of this disclosure.

### DETAILED DESCRIPTION

The present invention is directed to an article of apparel or footwear including a textile comprising a treated substrate, as disclosed in claims 1-6.

One or more aspects of the present disclosure are directed to aqueous solutions that can be used to make substrates such as an article that can inhibit or limit one or more sources of odor. In an aspect, the aqueous solution can include one or more components, where one of the components is an inhibiting agent that can function to inhibit or limit the sources of odor in an article such as a textile. In a particular aspect, the inhibiting agent can function as a lipid peroxidation inhibitor, an aldehyde inhibitor, and/or an antioxidant. The solution can also include one or more additional components such as: a stain release agent, a cross-linker, a softener, and an alkali metal salt catalyst. The substrate can be manufactured by exposing the aqueous solution to an untreated substrate to form a surface coating on the article and the article has the characteristic of inhibiting or limiting the sources of odor. In an aspect, the substrate can be an assembled article such as a shirt or can be a part of an article that is subsequently cut and assembled into an article. An example of a substrate includes a textile article, fully assembled or uncut and unassembled, including polyester (e.g., polyethylene terephthalate (PET)), and it is known that textiles including polyester (e.g., PET) retain odor more than other textiles. In this regard, the textile including polyester can be treated (before or after assembly) with the aqueous solution to form a surface coating on the textile and the treated textile has the characteristic of retaining less odor than textiles that are not treated with the aqueous solution.

In an aspect, the present disclosure provides for an aqueous solution comprising: aqueous solution comprising: a trehalose at about 0.01 to 10 weight % of the solution and one or more of the following: polyacrylic acid at about 0.01 to 5 weight % of the solution; sodium hypophosphite at about 0.01 to 10 weight % of the solution; and polyethylene glycol ester at about 0.01 to 6 weight % of the solution.

Another aspect of the present disclosure includes methods of treating a substrate (e.g., textile, polyesters) that includes exposing the substrate to the aqueous solution as described above or herein; drying the substrate; and curing the substrate to form a treated substrate.

An aspect of the present disclosure includes an article, comprising: a treated substrate having a surface coating comprising an inhibitor agent.

Now having describe aspect of the present disclosure generally, additional discussion regarding aspects will be described in greater detail.

This disclosure is not limited to particular aspects described, and as such may, of course, vary. The terminology used herein serves the purpose of describing particular aspects only, and is not intended to be limiting, since the scope of the present disclosure will be limited only by the appended claims.

Where a range of values is provided, each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure. Any recited method may be carried out in the order of events recited or in any other order that is logically possible.

Aspects of the present disclosure will employ, unless otherwise indicated, techniques of material science, chemistry, textiles, polymer chemistry, and the like, which are within the skill of the art. Such techniques are explained fully in the literature.

Unless otherwise defined, all technical and scientific terms used herein have the same meaning as commonly understood by one of ordinary skill in the art of microbiology, molecular biology, medicinal chemistry, and/or organic chemistry. Although methods and materials similar or equivalent to those described herein can be used in the practice or testing of the present disclosure, suitable methods and materials are described herein.

As used in the specification and the appended claims, the singular forms "a," "an," and "the" may include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

As described above, one or more aspects of the present disclosure provide aqueous solutions that can be used to make a surface coating or layer on a treated structure (*e.g*., a treated textile), where the surface coating has the characteristic of inhibiting or limiting one or more sources of odor. As used herein, "odor" refers to unpleasant or disagreeable smells associated with an article or textile. For example, the textile can have a malodorous smell as a result of activities (*e.g*., exercise, physical labor, strenuous activity, or the like) and/or residual smells after cleaning. Some materials such as polyesters retain odors more than other materials, so while these materials may provide comfort or other desirable characteristics, the odor associated with these materials is not desirable.

Odor can be formed in many ways and a number of mechanisms produce the malodorants. In short, the components in sweat (*e.g.,* water, proteins, lipids (*e.g.,* fatty acids), and other compounds), other skin secretions, or other bodily fluids can interact with bacteria naturally on the skin to break down these components to generate volatile malodorants compounds. The types of bacteria and the types of compounds that can be produced varies considerably, which makes addressing odor a complex problem.

The words "inhibit" or "inhibiting" do not mean that the source of odor must be inhibited 100%, rather the source of the odor can be reduced relative to an untreated substrate, article, or textile. For example, the amount of inhibition can include about 10% or more, about 15% or more, about 25% or more, about 30% or more, about 40% or more, about 50% or more, about 60% or more, about 70% or more, about 80% or more, about 90% or more, about 95% or more, about 99% or more, or about 100% inhibition relative to an untreated structure. In addition, the words "inhibit" or "inhibiting" mean reducing or hindering the ability of the odor sources (*e.g.,* chemical and/or biological) to produce or generate (*e.g*., via bacteria), directly or indirectly, the odor, which may include the chemicals associated, directly or indirectly, with the odor. The chemical or biochemical process can be disrupted at one or more stages to reduce the amount of odor causing chemicals or prevent the process from even occurring.

In an aspect, the structure can be the article or be a part of the article. In an aspect the article can include a textile, where the textile can be a woven, non-woven, braided, or knit textile. In an aspect, the textile can include fibers, yarns, or fabrics made of natural (*e.g.,* cotton, silk, wool, and the like) and/or synthetic fibers (*e.g.,* polyesters, polyamides, polyolefins, and the like). The synthetic material can include polyesters, polyolefins, halogenated polymers, polyamides, polyurethanes, polyaramids, as well as homopolymers, copolymers, or terepolymers in any combination of these.

In aspects, the textile can be made of one or more elements, where multiple elements can be sewed, welded, or otherwise associated to or with one another to form the textile. In an aspect, the textile can be treated with the aqueous solution at any stage of manufacture or use (e.g. during textile manufacture, to a manufactured textile or article, to elements of an article before assembly of an article, after assembly of an article, device, or apparel item). In another aspect, the fibers, yarns, or fabrics can be treated prior to making the part of the article.

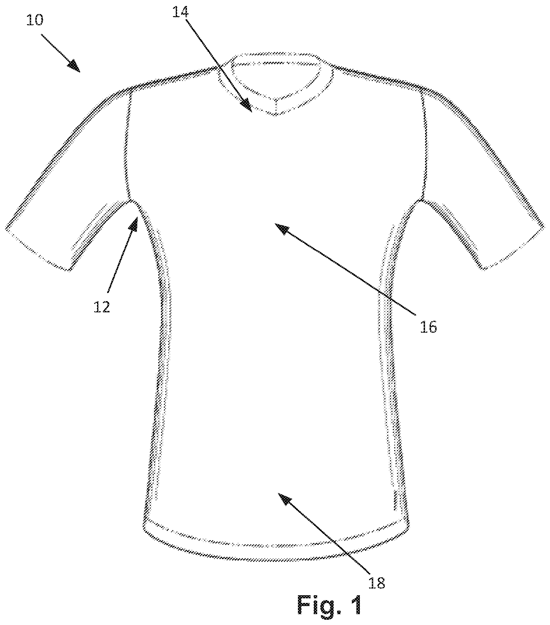

In the present invention, the textile is included in an article of apparel or footwear (*e*.*g*., shirts, jerseys, pants, shorts, gloves, glasses, socks, hats, caps, jackets, undergarments). Figure 1 illustrates a shirt 10, where one or more areas (*e.g*., 12, 14, 16, 18) of the shirt 10 can include a treated area with the aqueous solution.

In an aspect, the aqueous solution includes an inhibitor agent. The concentration of the inhibitor agent can be about 0.01 to about 15 wt% of the solution, about 0.01 to about 10 wt% of the solution, about 0.01 to about 5 wt% of the solution, about 0.01 to less than about 5 wt% of the solution, about 0.01 to less than 5 wt% of the solution (in this phrase "less than 5 wt%" does not include "less than about 5 wt%"), about 0.01 to about 4 wt% of the solution, about 0.01 to about 2 wt% of the solution, or about 0.01 to about 1 wt% of the solution. In each of the ranges above for the inhibitor agent, the lower range can also be any one of about 0.1, about 0.5, about 1, and about 2.

In an aspect, the inhibitor agent can function to disrupt or prevent one or more steps of one or more mechanisms that produce malodorants. The inhibitor agent can function in one or more of the following ways: as a lipid peroxidation inhibitor, as an aldehyde inhibitor, or as an antioxidant. Lipid peroxidation inhibition includes disrupting or preventing the oxidation of lipids (*e.g*. those excreted from the skin), which may include disrupting or preventing one or more steps in the mechanism for oxidizing lipids. Aldehyde inhibition includes disrupting or preventing the formation of aldehydes, which may include disrupting or preventing one or more steps in the mechanism for forming aldehydes. The antioxidant can disrupt or prevent the oxidation of compounds that can be made into malodorants, which may include disrupting or preventing one or more steps in the oxidation mechanism.

In the present invention, the polysaccharide includes trehalose.

The concentration of the trehalose can be about 0.01 to about 15 wt% of the solution, about 0.01 to about 10 wt% of the solution, about 0.01 to about 5 wt% of the solution, about 0.01 to less than about 5 wt% of the solution, about 0.01 to less than 5 wt% of the solution (in this phrase "less than 5 wt%" does not include "less than about 5 wt%"), about 0.01 to about 4 wt% of the solution, about 0.01 to about 2 wt% of the solution, or about 0.01 to about 1 wt% of the solution. In each of the ranges above for the inhibitor agent, the lower range can also be any one of about 0.1, about 0.5, about 1, and about 2.

In an aspect the aqueous solution can include one, two, or three of the following: a stain release agent, a cross-linker, a softener, and an alkali metal salt catalyst. In an aspect the aqueous solution can include each of the stain release agent, the cross-linker, the softener, and the alkali metal salt catalyst. In each aspect, one or more types of each of the stain release agent, the cross-linker, the softener, and/or the alkali metal salt catalyst can be present in the aqueous solution. In other words, the aqueous solution can include two or more types of cross-linkers, for example.

The stain release agent can function to reduce the ability of the treated substrate to stain and/or allow for the stain to be more easily removed from the substrate. In an aspect the stain release agent can include one or more of the following: a carboxymethyl cellulose salt, an oligomeric copolymers of terephthalic acid with ethylene glycol and polyethylene glycol, a carboxy based hydrophilic polymer, or a combination thereof. The concentration of the stain release agent can be about 0.01 to 5 weight % or about 0.5 to 5 weight % of the solution.

The crosslinker includes polyacrylic acid.

The concentration of the cross-linker can be about 0.01 to 5 weight % or 1 to 3 weight % of the solution.

The alkali metal salt catalyst can function to catalyze the cross-linking reaction(s). In an aspect the alkali metal salt catalyst can be selected from: alkali metal hypophosphites, alkali metal phosphites, alkali metal polyphosphonates, alkali metal phosphates, and alkali metal sulfonates. The hypophosphorous salt can be sodium hypophosphite. The concentration of the alkali metal salt catalyst can be about 0.01 to 10 weight % or about 1 to 5 weight % of the solution.

The softener can function to give the substrate a softer feel relative to the substrate without the softener. The softener can comprise a silicone, a fatty acid derivative, an aliphatic glycol ester, an aliphatic phthalate copolymer, a derivative of an aliphatic phthalate copolymer, or any combination thereof. In some aspects, the softener is a hydrophilic softener (*e.g*., a softener which, when applied to a substrate, results in the surface of the softener-treated substrate having a lower water contact angle than the surface of the untreated substrate). In an aspect the softener can be a polyethylene glycol ester (*e.g*., a molecular weight of about 300 to 100,000) or a polyethylene glycol ester derivative. In an aspect the softener can be a polyethylene terephthalate copolymer, or derivatives of polyethylene terephthalate. The concentration of the softener can be about 0.01 to 6 weight % or about 1 to 3 weight % of the solution.

Now having described the aqueous solution, methods of using the aqueous solution and treated structure are now described. In an aspect, the method of treating the substrate includes exposing the substrate to the aqueous solution. The substrate can be exposed in a number of ways, such as dipping the substrate in the aqueous solution, foaming the aqueous solution onto the substrate, spraying the aqueous solution onto the substrate, combinations thereof, and the like. In an example, the substrate is a textile and known methods for introducing a solution to a textile can be used to introduce the aqueous solution to the textile.

After an appropriate amount of time (e.g., about 2 seconds to a minute or more) for the substrate to be exposed to the aqueous solution, the substrate and aqueous solution are separated from one another and the substrate is dried. In an example, the textile fabric can have a wet pickup of about 50 to 100 weight %. Wet pickup is a measure of the amount of liquid taken up by a substrate when the substrate is dipped into the liquid. Commonly, a substrate is weighted when dry and then dipped into a bath of the liquid, run through a series of rollers to wring excess liquid from the substrate, and re-weighed to determine its wet weight. As used herein, the weight percent of wet pickup is determined by subtracting the dry weight of the substrate from the wet weight of the substrate, dividing that value by the dry weight of the substrate, and then multiplying that value by 100.

The substrate is then cured (*e.g*., the substrate is heated to a temperature of about 120 to 160° C for 30 seconds to 5 minutes or more) to form the treated substrate that includes a surface coating. In an example, the substrate is a textile fabric and the surface coating is on the fabric. Although not intending to be bound by theory, components of the aqueous solution and/or the substrate cross-link to form the surface coating. For example, the inhibitor agent, the cross-linker and/or the stain release agent, can cross-link with one another and/or with the terminal groups of the substrate. In an aspect, trehalose, polyacrylic acid, and/or carboxymethyl cellulose, can cross-link with one another and/or with the terminal groups of the substrate such as a PET substrate.

The surface coating can be about 10 nm to 10,000 µm. In an aspect, the surface coating can be applied so that it is uniformly distributed throughout the thickness of a fabric (*e.g*., on and among the fibers, yarns, fabric, etc.). In another aspect, the surface coating can be applied so that it is applied to select portions of the fabric (*e.g*., areas, sides, and the like) to achieve the desired odor reducing characteristics. In an aspect, the treated substrate can include a product of the methods described herein.

In an aspect, the treated substrate can include a surface coating that includes the inhibitor agent (*e.g*., trehalose) or compounds incorporating the inhibitor agent in a way that the inhibitor agent functions to inhibit odor. In an aspect, the inhibitor agent can be covalently bonded to a polymer that is attached to the substrate, such as the fabric of a textile. In addition or in the alternative, the inhibitor agent can be non-covalently incorporated into the substrate such as the fabric of a textile.

In an aspect, the surface coating may not cover all surfaces of the substrate and portions of the substrate may be free of the surface coating. In another aspect, the surface coating may cover a large portion of the substrate, for example, the substrate is a fabric of a textile and the fabric contacting the skin is coated or the surface coating is on portions of the fabric that contact specific areas of the individual (*e.g*., under arms area, groin area, and the like). In another aspect, the surface coating may cover all of the substrate, for example all surfaces of a textile shirt.

In an aspect, one or more other components can be included in the surface coating such as the stain release agent, the cross-linker, the softener, and the alkali metal salt catalyst. Similarly to the inhibitor agent, each of these components are present in the surface coating or are incorporated in the surface coating in a way that the each functions as they are intended. The surface coating can include one or more cross-linked groups that incorporate one or more of the components. The alkali metal salt catalyst may or may not be present in the surface coating since this component is used to catalyze the cross-linking reactions.

As stated above, the substrate, or precursor materials (*e.g*., fibers, yarn, etc.), can be treated with the aqueous solution. The treatment can be at any stage of manufacture and the treatment can be for the entire substrate or for individual elements used to make the substrate. For example, one or more areas of the textile to form the textile can be treated during manufacture of the textile, during manufacture of elements of textile before assembly, or after assembly of the textile. In one aspect, the textile can be treated after assembly of the elements used to form the textile.

In another aspect, the manufacturing of the textile, for example, can include one or more of the following actions: a) making the textile (*e.g*., a rolled good), b) treating a portion or all of the textile with the aqueous solution to prepare the treated textile (*e.g*., a treated rolled good), c) cutting the textile into individual elements, and d) assembling individual elements (*e.g*., sew or weld them together), treated and/or untreated elements, to form an article of apparel, where the order of b) and c) can be switched. In an aspect, a single party can perform each of these actions to manufacture the textile. In another aspect, two or more can independently perform these actions to manufacture the textile.

### EXAMPLES

For the following examples, unless otherwise specified, 100% polyester knit single jersey fabric of 145 grams per square meters, dyed black and ready for finish, was used for the treatment.

### Formulation of the aqueous solution and method of treating the textile

Treatment Solution A, containing the following chemicals was prepared: 2 wt% trehalose, 2 wt% sodium hypophosphite, 8 wt% polyacrylic acid (20% solution, MW 2000 - 10,000), and 3 wt% polyethylene glycol ester. The solution pH was adjusted with acid to 4-5. Fabric was dipped and padded in the solution and run between 2 rollers to achieve a 70-80 wt% wet pickup, then dried and cured at 140° C for 2 minutes.

### Testing procedures for Aldehyde Odor Inhibition

First, a small sample of substrate (2.25 cm²±0.1 cm²) was cut. For the purposes of the testing, the substrate was a polyester knit fabric textile product as described above. The sample was placed in an injection vial, lying flat on the bottom of the vial. The substrate was treated with aqueous Treatment Solution A, described above.

8 µL triolein was inserted by injection syringe through the sealing film into the vial flask on the sample. Triolein is used as a test compound as it is a compound which, when oxidized, breaks down into C5-C12 (or C5-C8) aldehydes. In the method, triolein is oxidized in the presence of untreated and treated substrate to evaluate the effect of the treatment on the level of aldehydes detected in the head space of the samples

3 µL of the internal standard component trimethylsilanol was then inserted onto the vial flask wall, taking care to avoid contact with the sample and the odor component.

The vial flask was kept at 180°C for 30 minutes using a heating plate with no agitation or stirring of the testing gas. After the 30 minutes of heating, a gastight syringe was inserted vertically into the center of the sealing film and 1 mL of odor testing gas sampled from inside the vial flask with the syringe.

The concentration of the testing gas was then sampled using gas-chromatography-mass spectrometry (GC-MS). The testing gas obtained from the vial flask was injected into the column of the GC, and the concentration of odor component chemicals were then detected by mass spectrometer. The peak area value of the MS were obtained as the value proportional to the concentration of the testing gas.

### Calculation of odor reduction rate

The following formula was used to calculate the rate of reduction of odor compounds from the samples: $ORR = \frac{\left(Sb-Sm\right)}{Sb} \times 100$

Where ORR is the odor reduction rate, as a percentage; Sm is the target C5-C12 aldehyde odor peak ratio of Mass Spectrum of the testing gas for the treated sample; and Sb is the target odor peak ratio of Mass Spectrum of the testing gas for the untreated sample.

To test for durability of the fabric treatment during laundering, all fabrics were washed in hot water (at 60°C), with a protocol according to AATCC method M6. Results of the Odor Reduction Rate test before and after are shown in Table 1 Treated polyester fabrics showed reduction of C6 to C8 aldehyde generation from triolein oxidation. Even after 25 wash and dry cycles, the treatment demonstrated its durable inhibition effectiveness.

**Table 1: Aldehyde reduction rates (ORR) of treated polyester fabrics**

| | Before wash | 5 washes | 25 washes |
|---|---|---|---|
| Pentanal | 37.86 | 32.05 | 23.3 |
| Hexanal | 27.07 | 23.04 | 9.5 |
| Heptanal | 31.63 | 33.11 | 16.4 |
| Octanal | 21.41 | 25.56 | 16.7 |
| n-Nonanal | 22.66 | 17.82 | 7.2 |
| 2-Nonenal | 23.37 | 25.67 | 15.78 |
| 2-Decenal | 21.58 | 36.83 | 21.54 |
| 2-Dodecenal | 21.58 | 30.53 | 24.6 |

### Aldehyde Odor Analysis of worn fabric samples

Bi-symmetrical t-shirts were made up for field wear trials. The left and right halves of each test t-shirt were formed from separate panels, so that all the fabric on one half of the t-shirt was treated, and all the fabric on the other half was the control fabric (untreated).

Wear testers wore these t-shirts for their daily workouts (4 - 12+ hours weekly) and washed them afterwards with their regular laundry. After the 25^{th} wear cycle (before the 25^{th} wash), all of the shirts were returned to the lab for analysis. The 25^{th} wash and dry were done in the lab using the same procedure as the fabric testing above. No deodorants, perfumes, softeners, dryer sheets, or bleach were used in the field wear trials.

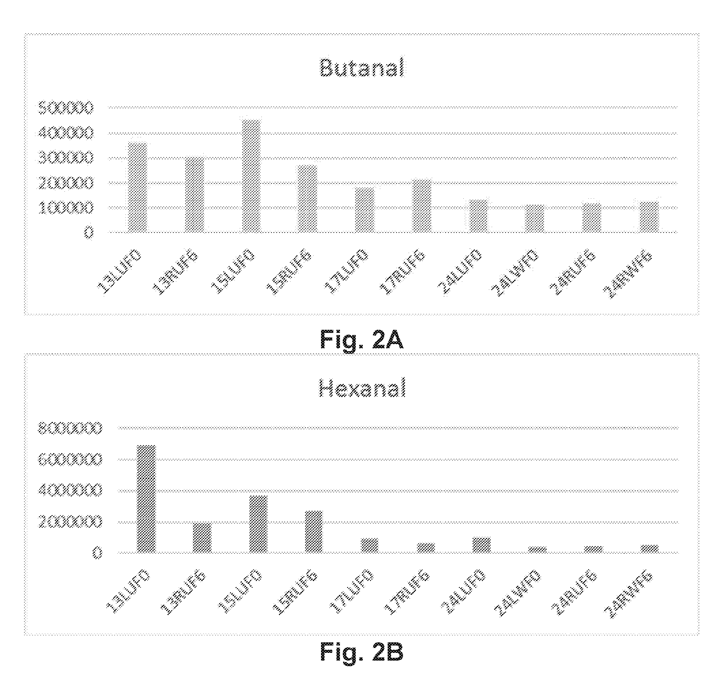

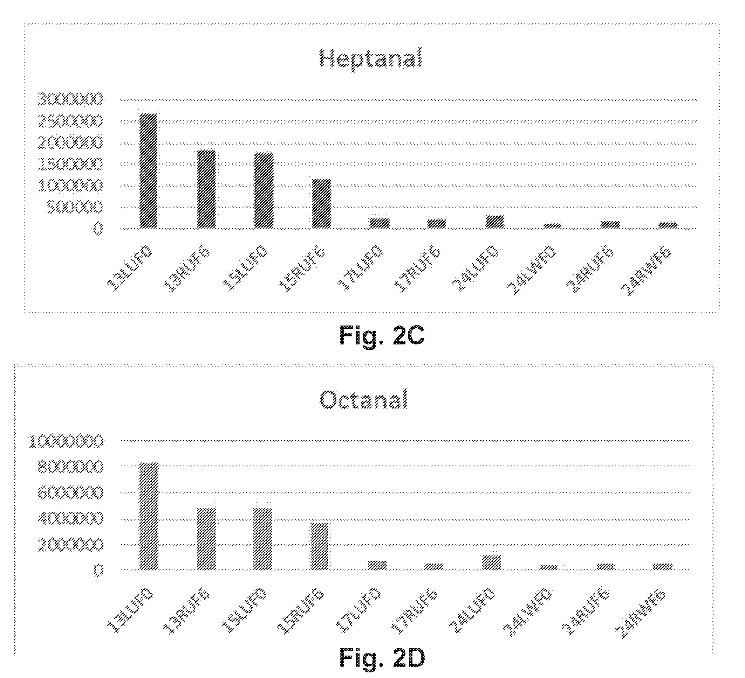

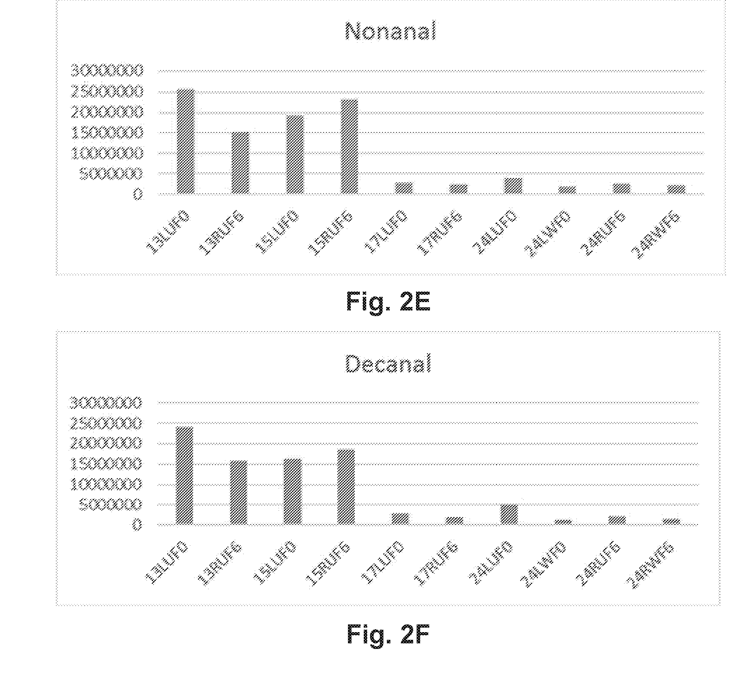

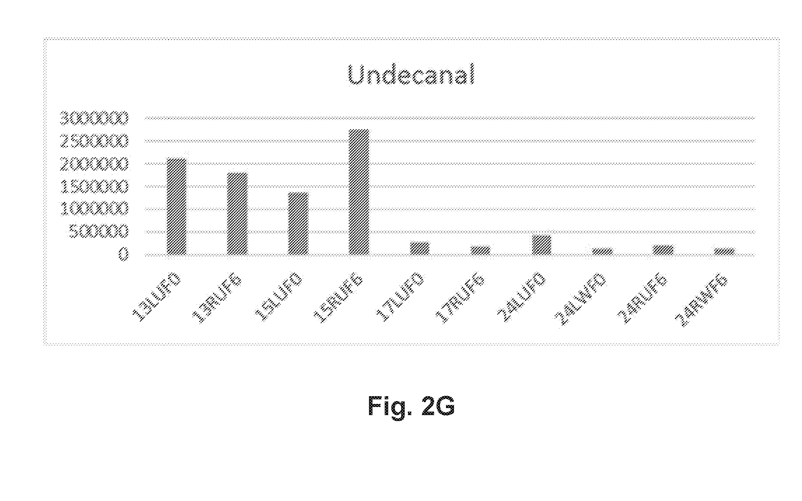

Ten fabric samples (8 unwashed and 2 washed) were analyzed by SPME-GC×GC-TOFMS using the methods described above. Solid-phase microextraction (SPME) extraction was performed at 30 °C for 24 hours. For the extraction, a divinylbenzene/carboxen/ polydimethylsiloxane (PDMS/CAR/DVB) fiber was used as the solid-phase substrate. Analysis was conducted on both the treated and untreated portions of the garment (Table 2). Results are shown in Figures 2A-G.

**Table 2 Sample code name explanations**

| **Code name** | **Sample Description** |
|---|---|
| 13LUF0 | Garment #1 from subject #1, untreated side of garment ("L" refers to left side), before 25th wash |
| 13RUF6 | Garment #1 from subject #1, treated side of garment ("R" refers to right side, before 25th wash |
| 15LUF0 | Garment #2 from subject #2, untreated side of garment, before 25th wash |
| 15RUF6 | Garment #2 from subject #2, treated side, before 25th wash |
| 17LUF0 | Garment #3 from subject #3, untreated side, before 25th wash |
| 17RUF6 | Garment #3 from subject #3, treated side, before 25th wash |
| 24LUF0 | Garment #4 from subject #4, untreated side, before 25th wash |
| 24RUF6 | Garment #4 from subject #4, treated side, before 25th wash |
| 24LWF0 | Garment #4 from subject #4, untreated side, after 25th wash |
| 24RWF6 | Garment #4 from subject #4, treated side, after 25th wash |

Figure 2A-G first shows different volatile aldehyde odor profiles/concentrations among individual wear testers.

When wearing garments, different individuals generate and deposit different amounts and types of odor-causing compounds on the garments. This is demonstrated above, as different concentrations of different volatile aldehydes were detected in the garments worn by different test subjects. With test subjects #1, #2, #3 and #4, the treatment was effective in reducing the amount of the more volatile aldehydes (butanal, hexanal, heptanal and octanal) detected on the garments after wear and before washing. With test subjects #1, #3 and #4, the treatment was effective in reducing the amount of the less volatile aldehydes (nonanal, decanal, and undecanal) detected on the garments after wear and before washing. The garments from test subject 4 were also tested after the 25th washing to determine whether or not the treatment affected the ability of laundering to remove volatile aldehydes from the treated side of the garment. After 25 washings, the levels of each of the aldehydes detected on the treated side of the garment were the same as or in some cases somewhat lower than the levels detected on the untreated side of the garment following the 25th washing. This demonstrates that, after repeated laundering, the presence of the treatment did not negatively affect the effectiveness of laundering in removing aldehydes from the garment.

Before the 25^{th} wash, all treated fabrics except #3 had lower butanal odor concentration than untreated fabrics (Figure 2A). For hexanal, heptanal, and octanal C6 - C8 saturated aliphatic volatile aldehydes, all treated fabrics showed inhibition effect compared to the untreated control fabrics (Figure 2B-D). For nonanal, decanal, and undecanal C9 - C11 less volatile aliphatic aldehydes, all treated fabrics except #2 demonstrated odor inhibition performance via lower aldehyde concentrations than untreated control fabrics. After the 25^{th} wash, most volatile C4 - C11 aldehydes were removed by washing and drying.

It should be emphasized that the above-described aspects of the present disclosure are merely possible examples of implementations, and are set forth only for a clear understanding of the principles of the disclosure. Many variations and modifications may be made to the above-described aspects of the disclosure without departing from

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt% to about 5 wt%, but also include individual concentrations (*e.g.,* 1%, 2%, 3%, and 4%) and the sub-ranges (*e.g.,* 0.5%, 1.1%, 2.2%, 3.3%, and 4.4%) within the indicated range. In an aspect, the term "about" can include traditional rounding according to significant figures of the numerical value. In addition, the phrase "about 'x' to 'y'" includes "about 'x' to about 'y'".

Many variations and modifications may be made to the above-described aspects. All such modifications and variations are intended to be included herein within the scope of this disclosure and protected by the following claims.

## Claims

1. An article of apparel or footwear including a textile comprising a treated substrate having a surface coating comprising trehalose and polyacrylic acid.

2. The article or component of claim 1, wherein the surface coating further comprises polyethylene glycol ester and/or sodium hypophosphite.

3. The article or component of claim 1 or claim 2, wherein the surface coating further comprises a stain release agent selected from a carboxymethyl cellulose salt, an oligomeric copolymer of terephthalic acid with ethylene glycol and polyethylene glycol, a carboxy based hydrophilic polymer, or a combination thereof.

4. The article or component of any preceding claim wherein the surface coating comprises trehalose, polyacrylic acid, and/or carboxymethyl cellulose cross-linked with one another and/or with the terminal groups of the substrate.

5. The article or component of claim 4, wherein the substrate is a polyethylene terephthalate (PET) substrate.

6. The article or component of any one of claims 1 to 4, wherein the textile includes polyester.

## Patentansprüche

1. Bekleidungs- oder Schuhwerkartikel, beinhaltend ein Textil, das ein behandeltes Substrat mit einer Oberflächenbeschichtung aufweist, die Trehalose und Polyacrylsäure umfasst.

2. Artikel oder Komponente nach Anspruch 1, wobei die Oberflächenbeschichtung ferner Polyethylenglykolester und/oder Natriumhypophosphit umfasst.

3. Artikel oder Komponente nach Anspruch 1 oder Anspruch 2, wobei die Oberflächenbeschichtung ferner ein Fleckablöse- bzw. -schutzmittel umfasst, das ausgewählt ist aus einem Carboxymethylcellulose-Salz, einem oligomeren Copolymer aus Terephthalsäure mit Ethylenglykol und Polyethylenglykol, einem carboxybasierten hydrophilen Polymer oder einer Kombination davon.

4. Artikel oder Komponente nach einem der vorhergehenden Ansprüche, wobei die Oberflächenbeschichtung Trehalose, Polyacrylsäure und/oder Carboxymethylcellulose umfasst, die miteinander und/oder mit den Endgruppen des Substrats vernetzt sind.

5. Artikel oder Komponente nach Anspruch 4, wobei das Substrat ein Polyethylenterephthalat(PET)-Substrat ist.

6. Artikel oder Komponente nach einem der Ansprüche 1 bis 4, wobei das Textil Polyester beinhaltet.

## Revendications

1. Article d'habillement ou chaussant incluant un textile comprenant un substrat traité ayant un revêtement de surface comprenant du tréhalose et de l'acide polyacrylique.

2. Article ou composant selon la revendication 1, dans lequel le revêtement de surface comprend en outre de l'ester de polyéthylène glycol et/ou de l'hypophosphite de sodium.

3. Article ou composant selon la revendication 1 ou revendication 2, dans lequel le revêtement de surface comprend en outre un agent d'aptitude au détachage sélectionné parmi un sel de carboxyméthylcellulose, un copolymère oligomère d'acide téréphtalique avec de l'éthylène glycol et du polyéthylène glycol, un polymère hydrophile à base de carboxy ou une combinaison de ceux-ci.

4. Article ou composant selon l'une quelconque des revendications précédentes, dans lequel le revêtement de surface comprend du tréhalose, de l'acide polyacrylique et/ou de la carboxyméthylcellulose réticulé(e) l'un avec l'autre et/ou avec les groupes de terminaison du substrat.

5. Article ou composant selon la revendication 4, dans lequel le substrat est un substrat de polyéthylène téréphtalate (PET).

6. Article ou composant selon l'une quelconque des revendications 1 à 4, dans lequel le textile inclut du polyester.
